# FASCICULE DE BREVET EUROPEEN

(11) **EP 1 567 094 B1**
(45) Date de publication et mention de la délivrance du brevet: **14.03.2007**
(21) Numéro de dépôt: 03796148.9
(22) Date de dépôt: 03.12.2003
(51) Int. Cl.: A61F 2/16, B65B 31/00

(54) **PROCEDE ET DISPOSITIF D'EMBALLAGE STERILE D'UNE LENTILLE INTRAOCULAIRE HYDROPHILE SOUPLE PRETE A L'EMPLOI**
VERFAHREN UND VORRICHTUNG ZUM VERPACKEN VON HYDROPHILEN, FLEXIBLEN, EINSATZBEREITEN INTRAOKULARLINSEN
METHOD AND DEVICE FOR STERILE PACKAGING OF A READY-TO-USE FLEXIBLE HYDROPHILIC INTRAOCULAR LENS

(30) Priorité: 04.12.2002 FR 0215294
(43) Date de publication de la demande: 31.08.2005
(73) Titulaire: Ophthalmopharma AG, 8001 Zurich (CH)
(72) Inventeur: MAURAN, Christian, F-31240 Saint Jean (FR); GUILLAUME, Mathieu, F-74940 Annecy-le-Vieux (FR)
(74) Mandataire: Cabinet BARRE LAFORGUE & associés
(86) Numéro de dépôt international: PCT/FR2003/003575
(87) Numéro de publication internationale: WO 2004/052241

(56) Documents cités:
- WO-A-98/20819
- US-A- 4 787 904
- US-A- 4 860 885
- US-A- 5 425 734
- US-A- 6 129 733
- US-A1- 2002 156 486

## Description

L'invention concerne un procédé et un dispositif pour l'emballage et la conservation à l'état stérile d'une lentille intraoculaire hydrophile souple prête à l'emploi, c'est-à-dire prête à être implantée par injection au travers d'une incision ménagée dans la paroi de l'oeil d'un patient.

Les lentilles intraoculaires souples présentent l'avantage de pouvoir être pliées, ce qui permet leur passage au travers d'incisions de petites dimensions. Pour réaliser ce pliage et l'injection de la lentille dans l'oeil, divers dispositifs ont été proposés. Par exemple, WO 9628121 ou WO 9615743 ont proposé, dans le cas de lentilles pouvant être conservées à sec (en matériaux non hydrophiles, tels les silicones), des dispositifs d'injection jetables comprenant une cartouche de pliage et un dispositif d'injection. La lentille peut être chargée dans la cartouche avant emballage et stérilisation à la vapeur dans un autoclave.

WO 98 20819 décrit aussi un dispositif d'emballage d'un kit d'injection d'une lentille non hydrophile, comprenant une chambre de stockage de la lentille à l'état non plié, un dispositif de transfert et pliage de la lentille dans une chambre d'un injecteur. L'ensemble est maintenu sur un plateau support lui-même enveloppé d'une enveloppe. Là encore, l'ensemble peut être stérilisé.

Néanmoins, de tels dispositifs ne sont pas compatibles avec les lentilles intraoculaires, dites hydrophiles, en matériau(x) hydrophile(s) nécessitant d'être maintenues à l'état hydraté pour leur conservation.

Dans tout le présent texte, on désigne par « lentille hydrophile » toute lentille formée en un matériau tel qu'elle nécessite d'être maintenue dans une solution liquide pour sa conservation avant emploi. Il peut s'agir par exemple des lentilles en matériaux désignés « hydrogel », « acrygel » ou « acrylique » (ce terme étant alors détourné de son sens commun), qui sont des PMMA (polyméthylméthacrylate) et/ou HEMA (hydroxyméthylméthacrylate), hydratés à plus de 16%, notamment entre 24% et 28%. US-4 787 904 décrit aussi divers exemples de matériaux pouvant être utilisés pour réaliser une lentille hydrophile.

Les lentilles hydrophiles présentent notamment l'avantage d'une bonne biocompatibilité. En particulier, elles sont compatibles avec l'humeur aqueuse naturelle du point de vue chimique, physique et optique (angle de contact approprié). Elles présentent en outre une grande souplesse, ce qui permet leur pliage et/ou leur roulage pour faciliter leur insertion à travers une incision de dimension réduite, notamment à travers l'incision réalisée pour introduire dans l'oeil le matériel nécessaire au traitement chirurgical préalable (par exemple incision de 3mm à 3,5mm pour l'ablation du cristallin par phaco-émulsification). De plus, elles présentent simultanément une bonne mémoire de forme, de sorte qu'elles reprennent leur forme initiale fonctionnelle après insertion dans l'oeil.

Mais, le problème qui se pose pour ces lentilles hydrophiles est justement celui du pliage et de leur manipulation au moment de l'acte chirurgical. US-4 787 904 proposait de conserver la lentille à l'état préplié dans le dispositif d'injection baignant dans une solution de conservation, le tout étant contenu dans une poche d'emballage souple. Cette solution ne peut néanmoins pas être utilisée en pratique et ce pour deux raisons principales. Tout d'abord, la lentille restant pliée sur une longue durée avant utilisation conserve nécessairement une mémoire de forme de l'état plié, et ne reprend donc plus sa forme initiale fonctionnelle parfaite après implantation.

De surcroît, un tel emballage ne peut pas être stérilisé à la vapeur (autoclave) après réalisation. En effet, la solution liquide dans la poche entraînerait une surpression conduisant à l'explosion de la poche. La réalisation d'un tel emballage n'est donc pas compatible avec les impératifs de stérilisation de la lentille et de son emballage.

Dès lors, jusqu'à maintenant, les lentilles hydrophiles sont conservées à plat dans des flacons rigides stérilisés de solution de conservation. Au moment de l'acte chirurgical, le chirurgien prélève la lentille à l'aide d'une pince, la plie (avec l'aide éventuelle d'un dispositif plieur) ou la place dans une cartouche de pliage/injection, ou dans un injecteur et l'injecte dans l'oeil. Or, toutes ces manipulations sont relativement complexes, délicates, augmentent les risques de contamination et d'endommagement de la lentille.

L'invention vise donc à pallier ces inconvénients, en proposant un procédé et un dispositif d'emballage d'une lentille intraoculaire hydrophile souple grâce auxquels :
- la lentille est conservée à plat dans une solution liquide de conservation, et n'est pliée qu'au moment de l'acte chirurgical,
- la stérilité de l'emballage et de la lentille est obtenue facilement et peut être garantie jusqu'à l'implantation, malgré la présence de la solution liquide de conservation,
- l'emballage est étanche et ne présente aucun risque de fuite de solution liquide de conservation, avant, pendant ou après la stérilisation,
- la lentille n'a pas à être manipulée lors de son implantation, ni pour son pliage, ni pour son injection.

L'invention vise de surcroît à proposer un procédé et un dispositif d'emballage simples et peu coûteux. En particulier, l'invention vise à proposer un dispositif d'emballage à usage unique, jetable après implantation de la lentille, compatible aux différents modèles de lentilles.

Pour ce faire, l'invention concerne un procédé d'emballage d'une lentille intraoculaire hydrophile souple dans lequel :
- on place la lentille sur un support d'injection comprenant une extrémité d'implantation par laquelle la lentille peut être glissée et éjectée en vue de son implantation intraoculaire, ce support d'injection étant adapté pour recevoir et porter la lentille et pour pouvoir être associé à un dispositif d'injection comprenant un piston pousseur apte à pousser la lentille sur le support d'injection vers l'extrémité d'implantation,
- on met la lentille et le support d'injection dans un emballage renfermant un volume de solution liquide de conservation de la lentille venant baigner la lentille et la maintenant hydratée,
caractérisé en ce que:
- on utilise un support d'injection adapté pour recevoir et porter la lentille à plat et pour pouvoir réaliser un pliage de la lentille avant que cette dernière ne soit éjectée via l'extrémité d'implantation,
- on place la lentille à plat sur le support d'injection, et on l'immerge dans un bain de solution liquide de conservation contenue dans un flacon rigide étanche aux liquides que l'on referme,
- on stérilise ensuite l'ensemble à la vapeur, notamment par passage en autoclave dans un bain de vapeur à haute température (supérieure à 130°C, -notamment 132°C- pendant plus de 20 min -notamment 21 min-).

Avantageusement et selon l'invention, avant la stérilisation, on place le flacon rigide (contenant la lentille et la solution, et fermé) dans une enveloppe d'emballage externe compatible avec une stérilisation à la vapeur.

L'invention s'étend à un dispositif pour la mise en oeuvre d'un procédé selon l'invention.

L'invention concerne ainsi également un dispositif d'emballage et de conservation à l'état stérile d'une lentille intraoculaire comprenant :
- un support d'injection comprenant une extrémité d'implantation par laquelle la lentille peut être glissée et éjectée en vue de son implantation intraoculaire, ce support d'injection étant adapté pour recevoir et porter la lentille et pour pouvoir être associé à un dispositif d'injection comprenant un piston pousseur apte à pousser la lentille sur le support d'injection vers l'extrémité d'implantation,
- une lentille intraoculaire hydrophile souple placée sur le support d'injection,
- un emballage renfermant au moins la lentille, le support d'injection et un volume de solution liquide de conservation de la lentille venant baigner la lentille et la maintenant hydratée,
caractérisé en ce que:
- ce support d'injection est adapté pour recevoir et porter la lentille à plat et pour pouvoir réaliser un pliage de la lentille avant que cette dernière ne soit éjectée via l'extrémité d'implantation,
- la lentille est portée à plat sur le support d'injection, et immergée dans un bain de solution liquide de conservation contenue dans un flacon rigide étanche aux liquides et fermé,
- l'ensemble est à l'état stérilisé.

Avantageusement et selon l'invention, le flacon rigide est renfermé dans une enveloppe d'emballage externe compatible avec une stérilisation à la vapeur et l'ensemble est à l'état stérilisé.

L'état stérilisé du dispositif peut être vérifié et garanti de façon connue en soi, par exemple par des marquages colorés de garantie de stérilisation qui se modifient si l'enveloppe est contaminée ou risque de l'être. Le dispositif d'emballage selon l'invention comprend au moins la lentille, le support d'injection et la solution liquide de conservation dans le flacon rigide. Il peut contenir d'autres organes ou éléments accessoires.

Avantageusement et selon l'invention, on utilise un support d'injection adapté pour réaliser un pliage par simple mouvement de translation imparti à la lentille lorsque cette dernière est poussée vers l'extrémité d'implantation. Ainsi, le support d'injection et de pliage est exempt de pièce mobile (seul le piston étant mobile), ce qui simplifie et fiabilise le dispositif d'emballage. Le pliage (ce terme englobant aussi l'enroulage) est obtenu grâce à des portées de guidage -notamment hélicoïdales ou fuselées- ménagées dans le support d'injection pour guider des portions de bordure de la lentille.

Dans une première variante de l'invention, on utilise un support d'injection porté amovible par un bouchon de fermeture du flacon rigide. Le support d'injection, ainsi que la lentille qu'il porte, est maintenu immergé dans la solution liquide dans le flacon grâce au bouchon. Lors de l'utilisation, on sépare le bouchon du flacon, puis on sépare le support d'injection du bouchon, puis on associe le support d'injection à un dispositif d'injection à piston pousseur. Ce faisant, il est à noter que la lentille n'est jamais manipulée, pincée, ni pliée par des outils ; on évite donc tout risque de détérioration ou de contamination.

Dans une deuxième variante préférentielle de l'invention, on utilise un support d'injection associé à un dispositif d'injection comprenant un corps cylindrique creux recevant le piston pousseur adapté pour pouvoir coulisser de façon étanche dans le corps cylindrique. En outre, le flacon rigide et le corps cylindrique sont adaptés pour pouvoir être fixés l'un à l'autre rigidement, de façon étanche, le support d'injection s'étendant dans la solution liquide de conservation dans le flacon rigide, mais de façon à pouvoir être dissociés l'un de l'autre en vue de l'utilisation du dispositif d'injection pour l'implantation de la lentille.

Ainsi, l'ensemble du dispositif nécessaire à l'implantation de la lentille par injection, y compris la lentille, le support d'injection et de pliage, et le dispositif d'injection à piston pousseur, sont incorporés dans le même emballage et stérilisés ensemble à la vapeur avec la solution liquide et le flacon rigide. Lors de l'utilisation, il suffit alors d'extraire le dispositif d'injection (portant le support d'injection et la lentille) hors de l'emballage, de le séparer du flacon rigide, et d'injecter la lentille, sans aucune manipulation supplémentaire. Les termes « corps cylindrique creux » désignent un corps dont la paroi interne est une forme générale de cylindre au sens mathématique du terme, c'est-à-dire dont la section droite n'est pas nécessairement circulaire (bien que cette forme, correspondant à un cylindre de révolution, soit préférentielle), et peut être par exemple elliptique ou autre. Le corps cylindrique creux et le piston pousseur sont de formes conjuguées pour assurer l'étanchéité aux liquides entre eux, y compris lors des mouvements de translation du piston pousseur dans le corps cylindrique creux. Ils forment ainsi un dispositif assimilable à une seringue.

Avantageusement et selon l'invention, le flacon rigide et le corps cylindrique sont fixés l'un à l'autre par vissage d'une extrémité du flacon rigide sur la paroi extérieure du corps cylindrique. Avantageusement et selon l'invention, un joint est prévu pour assurer l'étanchéité aux liquides entre le flacon rigide et la paroi extérieure du corps cylindrique.

Avantageusement et selon l'invention, le dispositif comprend des moyens formant butée axiale empêchant l'extraction intempestive du piston pousseur hors du corps cylindrique creux.

De la sorte, on évite toute extraction du piston hors du corps cylindrique du dispositif d'injection lors de la stérilisation ou en fin de stérilisation, sous l'effet de la forte pression régnant dans le flacon et dans le support d'injection du fait de cette stérilisation.

Avantageusement et selon l'invention, le corps cylindrique creux est adapté pour former la butée d'extrémité axiale empêchant l'extraction intempestive du piston pousseur hors du corps cylindrique creux.

Avantageusement et selon l'invention, le dispositif comprend un joint d'étanchéité adapté pour être interposé entre la butée d'extrémité axiale du corps cylindrique creux, et un bloc d'étanchéité du piston pousseur en position extrême rétracté dans le corps cylindrique creux. Ce joint d'étanchéité comprimé entre le bloc d'étanchéité et la butée axiale assure une étanchéité parfaite aux liquides, notamment lors de la stérilisation. En variante, cette butée axiale peut être formée par une paroi rigide de l'enveloppe d'emballage formant un logement de réception du dispositif d'injection.

Par ailleurs, avantageusement et selon l'invention, le dispositif est doté de moyens de verrouillage déverrouillables du piston pousseur en position extrême rétracté dans le corps cylindrique creux. De la sorte, on évite tout actionnement intempestif du piston pousseur qui risquerait d'endommager la lentille, avant utilisation du dispositif selon l'invention. Pour ce faire, un dispositif selon l'invention est avantageusement caractérisé en ce que le piston pousseur comprend une tige de manoeuvre non symétrique de révolution, en ce que le corps cylindrique creux présente une extrémité axiale dotée d'une lumière non symétrique de révolution, de formes conjuguées de celles de la tige de manoeuvre, et en ce que la tige de manoeuvre est montée de façon à pouvoir être tournée autour de son axe longitudinal entre une position verrouillée où elle ne peut pas pénétrer dans le lumière et une position déverrouillée où elle peut passer dans la lumière.

Par ailleurs, avantageusement et selon l'invention, le support d'injection comprend une douille d'adaptation formant un logement de réception de la lentille, cette douille étant adaptée pour pouvoir porter et recevoir différents modèles de lentilles, cette douille d'adaptation étant aussi adaptée pour pouvoir être montée dans une portion d'extrémité cylindrique du support d'injection. De la sorte, le dispositif selon l'invention peut être utilisé avec différents types de lentille, sans modification. Les coûts de fabrication sont ainsi réduits.

L'invention s'étend aussi à un procédé et à un dispositif d'emballage caractérisés en combinaison par tout ou partie des caractéristiques mentionnées ci-dessus ou ci-après.

D'autres buts, caractéristiques et avantages de l'invention apparaissent à la lecture de la description suivante qui se réfère aux figures annexées représentant des modes de réalisation de l'invention donnés uniquement à titre d'exemples non limitatifs, et dans lesquels :
- la figure 1 est une vue schématique en perspective d'un dispositif d'emballage selon une première variante de réalisation de l'invention,
- la figure 2 est une vue schématique en coupe axiale éclatée du dispositif de la figure 1, l'enveloppe externe n'étant pas représentée,
- les figures 3 et 4 sont des vues schématiques en coupe axiale selon deux plans de coupe différents du dispositif d'emballage selon une deuxième variante préférentielle de réalisation de l'invention, l'enveloppe externe n'étant pas représentée,
- la figure 5 est une vue schématique en perspective éclatée illustrant un dispositif d'emballage selon une troisième variante de l'invention semblable à la deuxième variante, mais avec un support d'injection comprenant une douille d'adaptation,
- la figure 6 est une coupe axiale schématique illustrant un détail (extrémité du dispositif d'injection, support d'injection avec douille et lentille, et flacon rigide) de la troisième variante de réalisation de l'invention.

Un dispositif d'emballage selon l'invention est destiné à l'emballage et à la conservation à l'état stérile d'une lentille intraoculaire 1 hydrophile souple prête à l'emploi. Une telle lentille 1 comprend une partie optique 2 et deux haptiques 3. Cette lentille 1 est placée sur un support d'injection 4 comprenant une extrémité d'implantation 5 de forme extérieure effilée de façon à pouvoir être introduite à travers une incision ménagée dans la paroi de l'oeil d'un patient. La dimension et la forme intérieure de l'extrémité d'implantation sont adaptées pour permettre le passage de la lentille 1 via cette extrémité d'implantation et l'éjection de la lentille 1 hors de l'extrémité d'implantation 5 dans la cavité interne de l'oeil.

En pratique, la section droite de l'extrémité d'implantation 5 peut être circulaire ou carrée, ou rectangulaire, ou plus généralement polygonale, et ce, en fonction notamment de la forme présentée par la lentille 1 à l'état plié au moment de l'éjection.

A l'opposé de l'extrémité d'implantation 5, le support d'injection 4 comprend un logement 6 de réception d'une lentille 1 à l'état non plié (à plat). Ce logement 6 de réception est ménagé à l'intérieur d'une portion d'extrémité globalement cylindrique 7 du support 4 d'injection et débouche axialement pour former une ouverture 8 par laquelle la lentille 1 peut être insérée à l'intérieur du logement de réception 6.

Entre le logement de réception 6 et l'extrémité d'implantation 5, le support d'injection 4 comprend un cône de pliage 9 comprenant un conduit axial interne 10 communiquant d'un côté avec le logement de réception 6, et de l'autre avec l'extrémité d'implantation 5, et incorporant des portées 11 de guidage/pliage adaptées pour guider des portions de bordure de la lentille 1 -notamment de sa partie optique 2- de telle sorte que cette lentille 1 se trouve pliée (ou enroulée) lorsqu'elle est déplacée axialement en translation à l'intérieur de ce conduit 10 depuis le logement de réception 6 jusqu'à l'extrémité d'implantation 5. La forme extérieure du cône 9 de pliage est de préférence globalement tronconique de façon à relier continûment la paroi extérieure de la portion d'extrémité cylindrique 7 et celle de l'extrémité d'implantation 5. Il est à noter néanmoins qu'en variante toute autre forme qu'un tronc de cône pourrait être envisagée, et notamment un tronc de pyramide, ou même une forme cylindrique.

Les portées 11 de guidage/pliage du conduit 10 du cône 9 de pliage sont formées par les parois latérales internes opposées du conduit qui se rapprochent l'une de l'autre depuis le logement de réception 6 jusqu'à l'extrémité d'implantation 5.

La portion d'extrémité cylindrique 7 du support d'injection 4 est de préférence dotée de lumières radiales 12 traversantes permettant d'alimenter le logement de réception 6 en solution liquide de conservation depuis l'extérieur du support d'injection 4.

La paroi externe 13 de la portion d'extrémité cylindrique 7 du support d'injection 4 comprend des tétons 15 de fixation en saillie radiale vers l'extérieur. Le dispositif d'injection 14 présente une extrémité cylindrique creuse 33 dotée de lumières 16 adaptées pour recevoir les tétons 15 du support d'injection de façon à former des moyens de fixation relatifs rapides par engagement élastique anti-retour du support d'injection 4 à l'extrémité d'un dispositif d'injection 14. Les tétons 15 sont en forme de pêne demi-tour et adaptés pour se déformer élastiquement radialement vers l'intérieur pour venir s'engager dans les lumières 16 lors de l'introduction axiale du support d'injection 4 dans l'extrémité 33 du dispositif d'injection 14.

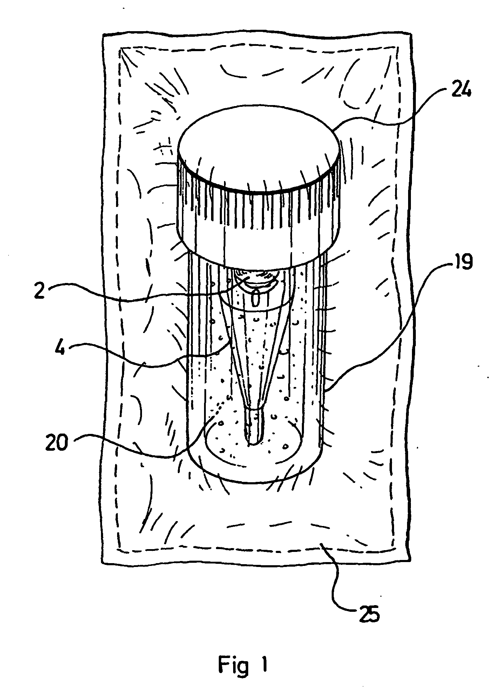

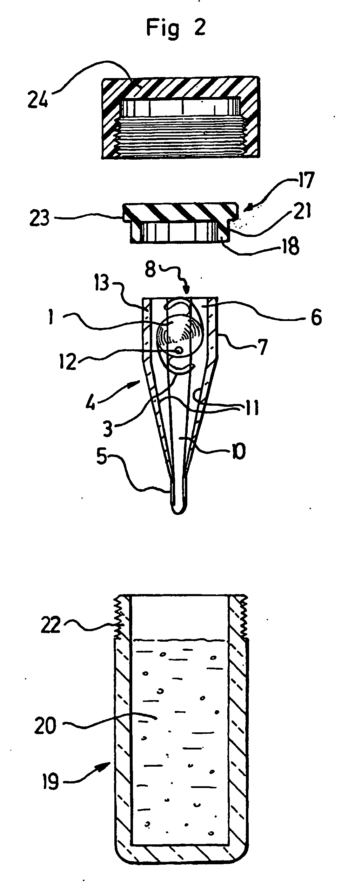

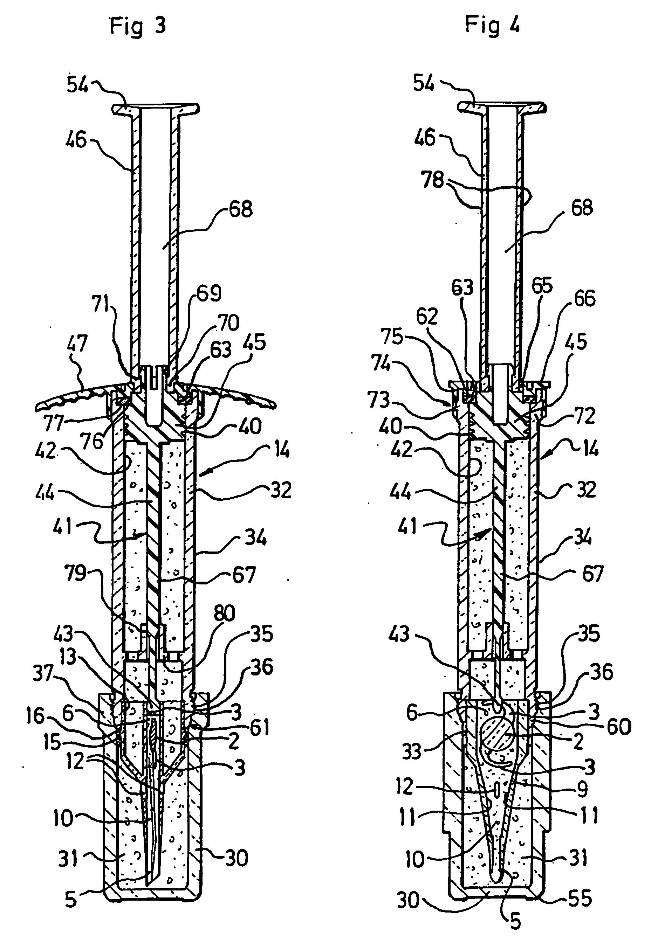

Dans les deux premières variantes des figures 1 à 4, le support d'injection 4 est formé d'une seule pièce monobloc constituant à la fois le logement de réception 6, les portées 11 de guidage/pliage, et l'extrémité d'implantation 5. Dans ces variantes, il faut néanmoins concevoir la forme exacte du logement de réception 6 en fonction de chaque modèle de lentille 1 qui doit être porté dans ce logement de réception 6. Or, en pratique, les lentilles 1 hydrophiles souples peuvent présenter différentes formes qui varient les unes des autres essentiellement par la forme et les dimensions de leurs haptiques. Les lentilles 1 varient également sensiblement en dimensions.

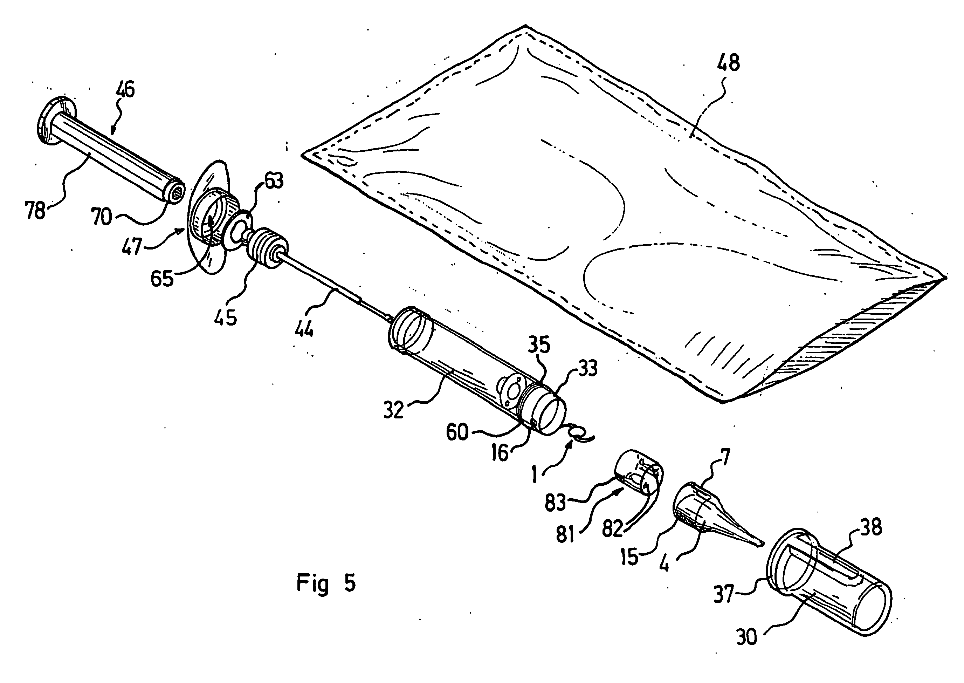

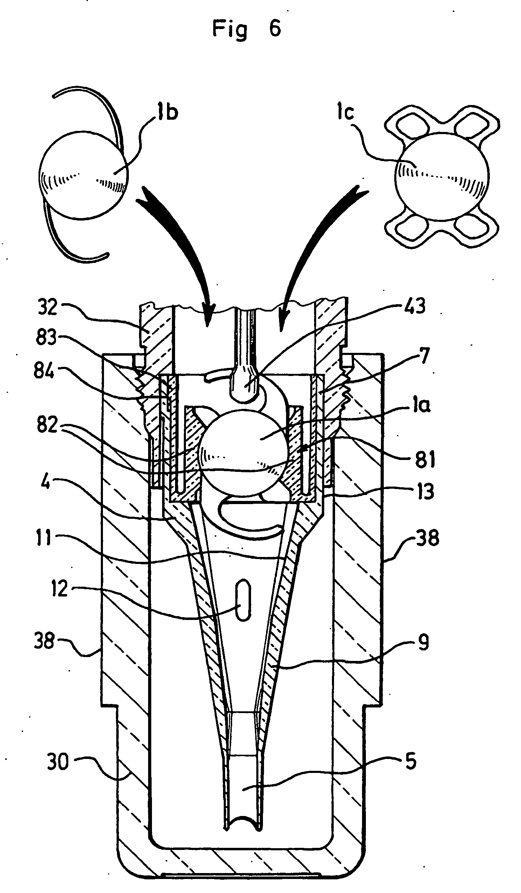

Pour éviter d'avoir à réaliser le support d'injection 4 en fonction de chaque modèle de lentille 1 à implanter, la troisième variante représentée figures 5 et 6 prévoit que le support d'injection 4 incorpore une douille d'adaptation 81 adaptée pour former le logement de réception 6 d'une lentille 1, 1a, 1b, 1c d'un modèle quelconque. Ainsi, cette douille d'adaptation 81 est adaptée pour porter et recevoir différents modèles de lentille 1, 1a, 1b, 1c. La douille 81 est aussi adaptée pour pouvoir être montée emmanchée dans la portion d'extrémité cylindrique 7 du support d'injection 4.

Cette douille d'adaptation 81 peut être formée d'un manchon cylindrique ouvert à ses deux extrémités axiales et présentant à l'intérieur deux pinces 82 latérales s'étendant axialement en regard l'une de l'autre pour recevoir la partie optique d'une lentille 1, 1a, 1b, 1c. Ces pinces 82 présentent des formes adaptées pour pouvoir recevoir et bloquer la partie optique de la lentille 1, 1a, 1b, 1c, tout en autorisant ultérieurement sa translation axiale en vue de l'injection, et ce, de façon compatible avec les différentes formes d'haptiques existantes. Sur la figure 6, la lentille la présente des haptiques épaisses courbes, la lentille 1b présente des haptiques fines courbes, et la lentille le est de type navette présentant quatre haptiques en boucle. Ces exemples ne sont pas limitatifs, d'autres formes pouvant être envisagées pour la lentille 1.

En pratique, on constate que la douille 81 d'adaptation représentée figure 6 permet de recevoir et porter ces différents modèles de lentille 1. Rien n'empêche néanmoins de prévoir plusieurs modèles de douille 81 pour recevoir différents types de lentille 1 et ce, de façon peu coûteuse en fabrication.

La douille 81 cylindrique peut être introduite et emmanchée dans la portion d'extrémité 7 du support 4. La douille 81 présente avantageusement une nervure externe 83 s'étendant parallèlement à l'axe de la douille 81 et adaptée pour venir s'engager dans une rainure 84 conjuguée de la portion d'extrémité 7 du support d'injection 4, de façon à repérer et imposer la position angulaire de la douille 81 par rapport au support d'injection 4.

Le support d'injection 4 peut être intégralement réalisé en matériau rigide synthétique, transparent ou non, adapté pour résister à la stérilisation à la vapeur, et par exemple en polycarbonate ou polyméthacrylate.

Dans la première variante de l'invention représentée, le support d'injection 4 est porté par un bouchon 17 en caoutchouc souple présentant une extension 18 cylindrique femelle adaptée pour pouvoir recevoir la portion d'extrémité 7 cylindrique du support d'injection 4 de telle sorte que le support d'injection 4 est alors porté par le bouchon 17. L'association du bouchon 17 au support d'injection 4 est néanmoins amovible en ce sens que le support d'injection 4 peut être dissocié du bouchon 17 par simple écartement relatif de ces deux pièces.

Le bouchon 17 est par ailleurs adapté pour pouvoir fermer de façon étanche un flacon 19 rigide contenant un bain de solution liquide 20 de conservation de la lentille 1 hydrophile souple, le support d'injection 4 et la lentille 1 étant immergés dans ce bain de solution liquide 20.

La paroi externe cylindrique 21 de l'extension 18 du bouchon 17 est adaptée pour s'insérer dans le goulot 22 (extrémité axiale ouverte) du flacon 19 et l'obturer de façon étanche. La paroi cylindrique 21 est prolongée à l'extérieur du goulot 22 par une portion 23 en forme de disque venant couvrir le chant d'extrémité du goulot 22.

De préférence, ce bouchon 17 en caoutchouc souple est lui-même recouvert par un capuchon rigide 24 de fermeture adapté pour refermer le tout de façon étanche et pour pouvoir se visser sur le flacon rigide 19, le bouchon 17 étant serré et maintenu en place entre le goulot 22 et le capuchon 24. Le capuchon rigide 24 est doté d'un taraudage adapté pour coopérer avec un filetage conjugué de la paroi externe du goulot 22 du flacon rigide. Le flacon rigide 19 est de préférence en verre ou en matière synthétique, transparente ou non, adaptée pour pouvoir résister à la stérilisation à la vapeur, notamment en polycarbonate ou en polyméthacrylate.

La solution liquide 20 placée à l'intérieur du flacon rigide 19 ne remplit pas intégralement le volume interne du flacon rigide 19, y compris après insertion du support d'injection 4 et fermeture du flacon rigide 19. En effet, un volume d'air suffisant doit être ménagé à l'intérieur du flacon rigide 19 pour permettre d'absorber les variations de pression induites au cours de la stérilisation à la vapeur réalisée ultérieurement. La fermeture étanche du flacon rigide 19 réalisée par le bouchon 17 et le capuchon 24 est étanche aux liquides mais non totalement hermétique aux gaz, et en particulier aux gaz sous pression tels que la vapeur d'eau.

Le flacon rigide 19 comprenant le support d'injection 4 avec une lentille 1 est lui-même renfermé à l'intérieur d'une enveloppe souple d'emballage externe compatible avec une stérilisation à la vapeur. De telles enveloppes souples de suremballage externe sont bien connues en elles-même, notamment pour le conditionnement des instruments chirurgicaux. Elle est adaptée pour laisser passer librement la vapeur d'eau à l'intérieur de la cavité qu'elle délimite mais pour protéger ultérieurement son contenu de toute possibilité de contamination bactérienne et est étanche aux liquides.

L'ensemble du dispositif d'emballage ainsi formé contient une lentille hydrophile souple non pliée, prête à l'emploi, à l'intérieur du support d'injection 4, et cette lentille 1 baigne dans la solution liquide 20 de conservation à l'intérieur du flacon 19. L'ensemble peut être stérilisé à la vapeur par passage dans un autoclave, à une pression, une température, et pendant une durée adaptée pour permettre la stérilisation de l'enveloppe 25, du flacon 19, du support d'injection 4 et de la lentille 1.

Bien sûr, il est préférable, avant de placer la lentille 1 dans un tel dispositif d'emballage, de s'assurer que l'ensemble des différents éléments de ce dispositif d'emballage soient eux-mêmes stérilisés. L'emballage est également de préférence réalisé dans des conditions aussi stérile que possible, selon les normes applicables.

Après stérilisation à la vapeur, l'enveloppe 25 peut être dotée, de façon connue en soi, d'un ou plusieurs marquage(s) coloré(s) de garantie de l'état stérile, connu(s) en eux-même.

Pour l'utilisation d'une lentille 1 ainsi emballée, il suffit d'ouvrir l'enveloppe 25 souple pour extraire le flacon 19, d'ouvrir le capuchon 24 rigide, d'ôter le bouchon 17, de séparer le support d'injection 4 du bouchon 17 et de connecter ce support d'injection 4 à un dispositif d'injection 14, lui-même préalablement stérilisé. Comme on le voit, l'ensemble peut être réalisé alors que tous les éléments sont à l'état stérile sans manipulation spécifique de la lentille 1, elle-même déjà en place dans son support d'injection 4. Le dispositif d'injection 14 et le support d'injection 4 peuvent être utilisés immédiatement pour l'implantation de la lentille 1 à travers une incision ménagée dans l'oeil du patient.

Dans la deuxième variante préférentielle de l'invention représentée, le support d'injection 4 se présente préalablement fixé au dispositif d'injection 14 qui fait office de bouchon pour un flacon rigide 30 contenant un bain de solution liquide 31 de conservation de la lentille 1. Autrement dit, le flacon rigide 30 vient coiffer et fermer, de façon étanche aux liquides, l'extrémité d'implantation 5 du support d'injection 4.

Le dispositif d'injection 14 comprend un corps cylindrique creux 32 dont une extrémité forme l'extrémité axiale 33 cylindrique dotée des lumières 16 recevant les tétons 15 de fixation du support d'injection 4. La paroi externe cylindrique 34 de cette extrémité 33 présente un filetage 35 externe adapté pour pouvoir coopérer avec un taraudage interne 36 ménagé dans le goulot 37 du flacon 30. Dans l'exemple représenté, le flacon 30 est cylindrique et son goulot 37 est formé par l'extrémité axiale ouverte débouchante de ce flacon cylindrique, c'est-à-dire au même diamètre que le reste du flacon. Le filetage 35 et le taraudage 36 sont adaptés pour réaliser un blocage en fin de vissage et de serrage et, entre eux, une étanchéité aux liquides à l'état vissé. Ils peuvent ainsi présenter des pas variables autobloquants tels que ceux connus sous la dénomination « filetages gaz ». Pour assurer l'étanchéité aux liquides entre la paroi externe cylindrique 34 et le flacon 30, il est prévu en outre une portée tronconique mâle 60 à l'extrémité de la paroi externe 34 et une portée tronconique femelle 61 conjuguée dans le goulot 37 du flacon 30, ces portées 60, 61 étant adaptées pour venir en contact l'une de l'autre en fin de vissage. En variante non représentée, un joint d'étanchéité peut être prévu entre les portées 60, 61 ou à un autre endroit du montage.

La partie du support d'injection 4 s'étendant axialement hors du dispositif d'injection 14 après fixation s'étend intégralement à l'intérieur du volume du flacon rigide 30. Pour faciliter le vissage et le dévissage du flacon 30, ce dernier est avantageusement pourvu de nervures 38 externes en saillie.

De la sorte, le flacon rigide 30 peut être fixé rigidement et de façon étanche au corps cylindrique creux 32 par simple vissage. Néanmoins, il est également possible ultérieurement de dévisser le flacon rigide 30 du corps cylindrique 32 de façon à dissocier ce flacon rigide 30 du support d'injection 4 et du dispositif d'injection 14 en vue de l'injection de la lentille 1.

Le corps cylindrique creux 32 incorpore un piston pousseur 41 comprenant une tige 44 d'injection s'étendant axialement à l'intérieur d'un alésage 42 axial de ce corps cylindrique 32. Le piston pousseur 41 comprend une extrémité axiale 43 d'éjection adaptée pour pouvoir venir au contact de la lentille 1 placée dans le logement de réception 6 et pousser cette lentille 1 axialement à travers le cône de pliage 9, puis à travers l'extrémité d'implantation 5, jusqu'à ce que cette extrémité 43 d'éjection débouche elle-même hors de l'extrémité d'implantation 5 en vue d'une implantation correcte de la lentille 1 dans la cavité interne de l'oeil. A l'état emballé, le piston pousseur 41 est néanmoins rétracté à l'intérieur du corps cylindrique creux 32, sans que son extrémité 43 d'éjection ne vienne au contact de la lentille 1.

L'extrémité d'éjection 43 du piston pousseur 41 est formée à l'extrémité de la tige 44 d'injection de ce piston pousseur 41 dont la longueur est adaptée pour pouvoir être insérée intégralement à l'intérieur du conduit interne ménagé dans le support d'injection 4, c'est-à-dire dans le logement de réception 6, le cône de pliage 9 et l'extrémité d'implantation 5. Autrement dit, si L1 est la longueur de la tige 44 d'injection du piston pousseur et L2 est la longueur du conduit interne du support d'injection 4 depuis l'ouverture 8 du logement de réception 6 jusqu'à l'extrémité d'implantation 5, il faut s'assurer que :
L1> L2 + ε, ε étant une longueur suffisante pour assurer la sortie de la deuxième haptique de la lentille 1 alors que la tige 44 d'injection est repoussée intégralement à l'intérieur du support d'injection 4.

L'extrémité d'éjection 43 du piston pousseur 41 est de préférence en forme concave ou de fourche pour coopérer avec la lentille 1.

A l'opposé de l'extrémité d'éjection 43, la tige 44 d'injection du piston pousseur 41 est solidaire d'un bloc d'étanchéité 45 adapté pour fermer transversalement de façon étanche l'alésage axial 42 du corps cylindrique creux 32. Ce bloc d'étanchéité 45 peut être réalisé à la façon d'un piston de seringue en matière synthétique souple avec plusieurs lèvres ou joints toriques 40, sa section transversale étant conjuguée de celle de l'alésage 42. Le piston pousseur 41 est prolongé au-delà du bloc d'étanchéité 45 à l'opposé de la tige 44 d'injection par une tige rigide 46 de manoeuvre qui s'étend axialement à l'extérieur du corps cylindrique creux 32, et peut être repoussée axialement à l'intérieur de l'alésage 42 en vue de l'implantation de la lentille 1. La longueur du corps cylindrique creux 32 correspond à celle de la tige de manoeuvre 46 de façon à permettre les manoeuvres et les mouvements en translation du piston pousseur 41 dans le corps cylindrique 32 en vue de l'implantation comme indiqué ci-dessus.

L'extrémité 72 du corps cylindrique creux 32 à l'opposé de l'extrémité rétrécie 33 présente, à la façon d'une seringue, une couronne 47 de manoeuvre s'étendant radialement en saillie pour faciliter le poussage de la tige de manoeuvre 46 du piston pousseur 41.

Dans le mode de réalisation représenté sur les figures, le piston pousseur 41 est formé de deux pièces distinctes assemblées axialement l'une à l'autre, à savoir, une première pièce 67 formant la tige 44 d'injection du piston pousseur 41 et le bloc d'étanchéité 45, et une deuxième pièce 68 formant la tige de manoeuvre 46. Pour l'assemblage de ces deux pièces 67, 68 l'une à l'autre, le bloc d'étanchéité 45 présente, à l'opposé de la tige 44 d'injection, un ergot 69 d'encliquetage fendu, s'étendant axialement, et l'extrémité 70 de la tige de manoeuvre 46 est ouverte et dotée d'un épaulement 71 de verrouillage sur l'ergot d'encliquetage 69.

En outre, le corps cylindrique creux 32 est également formé de deux pièces distinctes, à savoir une première pièce principale constituant le corps cylindrique creux 32, et une deuxième pièce 66 d'extrémité axiale refermant le corps cylindrique creux à son extrémité axiale 72 opposée à son extrémité 33 recevant le support d'injection 4.

Lorsque la pièce d'extrémité 66 n'est pas en place, la première pièce 67 du piston pousseur 41 peut être introduite à l'intérieur de l'alésage 42 du corps cylindrique creux 32. A la fin de cette introduction, la pièce d'extrémité 66 peut être placée au bout du corps cylindrique 32 pour venir le refermer. Là encore, la pièce d'extrémité 66 peut être assemblée à l'extrémité 72 du corps cylindrique creux, par simple engagement élastique et verrouillage, par exemple grâce à des tétons 73 en forme de pênes demi-tour ménagés en saillie radiale vers l'extérieur de la paroi 34 du corps cylindrique creux 32, et à des lumières 74 conjuguées ménagées dans une jupe 75 de la pièce d'extrémité 66 pour recevoir les tétons 73.

La pièce d'extrémité 66 forme également la couronne de manoeuvre 47.

De surcroît, la pièce d'extrémité 66 est adaptée pour former une butée d'extrémité axiale 62 empêchant l'extraction intempestive du piston pousseur 41, c'est-à-dire de la tige 44 d'injection et du bloc d'étanchéité 45, hors du corps cylindrique creux 32. Cette butée d'extrémité axiale 62 est formée d'une portée en forme de couronne de la pièce 66 adaptée pour recevoir axialement une portée en forme de couronne 76 en regard du bloc d'étanchéité 45. Un joint d'étanchéité annulaire 63 est avantageusement interposé entre la butée d'extrémité axiale 62 et le bloc d'étanchéité 45. Ce joint 63 peut être bloqué entre la butée 62 formée par la pièce 66 et un épaulement 77 de la paroi du corps cylindrique creux 32. Ainsi, lorsque le bloc d'étanchéité 45 est repoussé axialement vers l'extrémité 72 du corps cylindrique creux sous l'effet de la pression du liquide régnant à l'intérieur de l'alésage 42 (notamment lors de la stérilisation à la vapeur), le bloc d'étanchéité 45 vient comprimer le joint d'étanchéité 63 qui assure une parfaite étanchéité et empêche toute fuite de liquide.

La pièce 66 d'extrémité refermant le corps cylindrique creux est par ailleurs dotée d'une lumière 65 axiale centrale pour le passage de la tige de manoeuvre 46 lorsque cette dernière est poussée en vue de l'injection de la lentille. Avantageusement, cette lumière 65 a une section droite transversale qui n'est pas symétrique de révolution. Par exemple, elle présente globalement la forme d'un cercle coupé par deux cordes diamétralement opposées formées par des parois planes en regard. L'extrémité 70 de la pièce 68 formant la tige de manoeuvre 46 présente des dimensions radiales inférieures à la distance entre les deux parois planes en regard de la lumière 65, de sorte que cette extrémité 70 peut librement tourillonner à l'intérieur de la lumière 65. Par contre, la partie courante principale de la pièce 68 formant la tige de manoeuvre 46 présente une section droite transversale non symétrique de révolution, de formes et dimensions conjuguées de celle de la lumière 65 par laquelle elle doit coulisser. Dans l'exemple représenté, la tige de manoeuvre 46 présente donc deux méplats 78 diamétralement opposés. Lorsque les méplats 78 sont axialement alignés avec les parois planes en regard de la lumière 65, la tige 46 peut être introduite à travers la lumière 65 et le piston pousseur 41 peut être poussé en vue de l'injection de la lentille. Au contraire, lorsque les méplats 78 de la tige de manoeuvre 46 ne sont pas alignés avec les parois planes de la lumière 65, par rotation de la tige de manoeuvre 46 autour de son axe longitudinal et autour de l'ergot 69, la tige de manoeuvre 46, à l'extérieur du corps cylindrique creux et de la pièce 66 d'extrémité, ne peut pas être enfoncée à l'intérieur de la lumière 65. On réalise ainsi des moyens de verrouillage du piston pousseur 41 en position extrême rétractée dans le corps cylindrique creux 32. Mais ces moyens de verrouillage sont déverrouillables par simple rotation de la tige 46. Ces moyens de verrouillage empêchent l'actionnement intempestif du piston pousseur 41 avant l'utilisation, notamment lorsque l'ensemble est emballé et manipulé pour son transport.

Par ailleurs, la tige 44 d'injection du piston pousseur 41 de plus faible diamètre que le bloc d'étanchéité 45, est avantageusement guidée à l'intérieur de l'alésage 42, du côté de l'extrémité 33 du corps 32, par un guide cylindrique 79 de formes et de dimensions conjuguées de celles de la tige 44. Ce guide 79 est relié à la paroi 34 du corps cylindrique creux 32 par un flasque 80 doté de lumières permettant le passage du liquide lors du déplacement du piston. Pour empêcher la rotation de la tige 44 et du bloc d'étanchéité 45 autour de son axe, avantageusement, la section droite transversale de la tige 44 ainsi que celle du guide 79 ne sont pas symétriques de révolution, et sont par exemple carrées.

Le dispositif d'injection 14 ainsi formé est assimilable à une seringue dont le bloc d'étanchéité est prolongé par la tige 44 d'injection.

Ce dispositif d'injection 14 est intégralement formé en une matière adaptée pour pouvoir résister à la stérilisation à la vapeur, par exemple en polycarbonate ou en polyméthacrylate (PMMA).

Pour l'emballage d'une lentille 1, on place cette lentille 1 à plat dans le logement de réception 6 d'un support d'injection 4, on fixe le support d'injection 4 à l'extrémité du corps cylindrique 32 du dispositif d'injection 14, on introduit le support d'injection 4 et l'extrémité 33 du corps cylindrique creux 32 dans le flacon rigide 30 en vissant ce dernier sur la paroi externe du corps cylindrique creux 32 jusqu'à obtenir l'étanchéité aux liquides. Préalablement, le flacon rigide 30 contient une quantité de solution liquide de conservation adaptée pour qu'après mise en place du flacon rigide 30 sur le corps cylindrique creux 32, la lentille 1 baigne dans la solution liquide 31. Egalement, il est à noter qu'avant mise en place dans le flacon rigide 30, une quantité de solution liquide peut être introduite à l'intérieur du conduit du support d'injection 4, et de l'alésage 42 du corps cylindrique 32. L'ensemble baigne alors dans la solution liquide de conservation.

En effet, l'étanchéité aux liquides du flacon rigide 30 refermé par le dispositif d'injection 14 est obtenue d'une part, entre le corps cylindrique creux 32 et le goulot 37 du flacon via le taraudage 36 et le filetage 35, et d'autre part, entre le bloc d'étanchéité 45 du piston pousseur 41 et le corps cylindrique creux 32 grâce aux lèvres ou joints 40 et au joint 63. Ainsi, c'est le dispositif d'injection 14 lui-même qui fait office de bouchon pour le flacon rigide 30. On peut aussi considérer à l'inverse que c'est le flacon rigide 30 qui sert de bouchon pour le dispositif d'injection 14.

Après mise en place et assemblage du flacon rigide 30 et du dispositif d'injection 14 avec le support d'injection 4 dans le flacon rigide 30, l'ensemble peut être placé dans une enveloppe d'emballage 48 souple, puis stérilisé à la vapeur à une température, une pression, et pendant une durée, adaptées pour obtenir une stérilisation. En fin de stérilisation, aucun risque n'existe que le piston pousseur 41 ne soit éjecté hors du corps cylindrique 32 sous l'effet des pressions différentielles.

Pour l'utilisation d'un dispositif d'emballage conforme à cette deuxième variante de réalisation de l'invention, il suffit d'extraire l'ensemble formé par le dispositif d'injection 14 et le flacon rigide 30 hors de l'enveloppe 48, de dévisser le flacon 30 du corps cylindrique 32 de façon à en extraire le support d'injection 4 portant la lentille 1, puis, directement, d'utiliser le dispositif d'injection portant le support d'injection 4 pour implanter la lentille 1. Comme on le voit, aucune manipulation de la lentille 1 n'est nécessaire et celle-ci est parfaitement bien conservée à l'intérieur de la solution liquide contenue dans le flacon rigide 30, à l'état stérile.

L'invention peut faire l'objet de nombreuses variantes de réalisation par rapport aux modes de réalisation préférentiels décrits ci-dessus et représentés sur les figures qui peuvent aussi être combinés. Les formes spécifiques des différents éléments et le choix des matériaux, les dimensions relatives peuvent être différentes de celles décrites et représentées.

L'invention permet d'obtenir un dispositif prêt à l'emploi parfaitement stérile évitant toute manipulation de la lentille, cette dernière étant maintenue à l'état parfaitement stérile ainsi que le dispositif d'injection 14 et le support d'injection 4.

## Revendications

1. Procédé d'emballage d'une lentille (1) intraoculaire hydrophile souple dans lequel :
- on place la lentille (1) sur un support d'injection (4) comprenant une extrémité d'implantation (5) par laquelle la lentille (1) peut être glissée et éjectée en vue de son implantation intraoculaire, ce support d'injection (4) étant adapté pour recevoir et porter la lentille (1) et pour pouvoir être associé à un dispositif d'injection (14) comprenant un piston pousseur (41) apte à pousser la lentille (1) sur le support d'injection (4) vers l'extrémité d'implantation (5),
- on met la lentille (1) et le support d'injection (4) dans un emballage renfermant un volume de solution liquide de conservation de la lentille (1) venant baigner la lentille et la maintenant hydratée,
**caractérisé en ce que** :
- on utilise un support d'injection (4) adapté pour recevoir et porter la lentille (1) à plat et pour pouvoir réaliser un pliage de la lentille (1) avant que cette dernière ne soit éjectée via l'extrémité d'implantation (5),
- on place la lentille (1) à plat sur le support d'injection (4), et on l'immerge dans un bain de solution liquide (20, 31) de conservation contenue dans un flacon rigide (19, 30) étanche aux liquides que l'on referme,
- on stérilise ensuite l'ensemble à la vapeur.

2. Procédé selon la revendication 1, **caractérisé en ce qu'**avant stérilisation on place le flacon rigide (19, 30) dans une enveloppe d'emballage (25, 48) externe compatible avec une stérilisation à la vapeur.

3. Procédé selon l'une des revendications 1 ou 2, **caractérisé en ce qu'**on utilise un support d'injection (4) adapté pour réaliser un pliage par simple mouvement de translation imparti à la lentille (1) lorsque cette dernière est poussée vers l'extrémité d'implantation (5).

4. Procédé selon l'une des revendications 1 à 3, **caractérisé en ce qu'**on utilise un support d'injection (4) porté amovible par un bouchon (17) de fermeture du flacon rigide (19).

5. Procédé selon l'une des revendications 1 à 3, **caractérisé en ce qu'**on utilise un support d'injection (4) associé à un dispositif d'injection (14) comprenant un corps cylindrique (32) creux recevant le piston pousseur (41) adapté pour pouvoir coulisser de façon étanche dans le corps cylindrique (32), et **en ce que** le flacon rigide (30) et le corps cylindrique (32) sont adaptés pour pouvoir être fixés l'un à l'autre rigidement, de façon étanche, le support d'injection (4) s'étendant dans la solution liquide de conservation dans le flacon rigide (30), mais de façon à pouvoir être dissociés l'un de l'autre en vue de l'utilisation du dispositif d'injection (14) pour l'implantation de la lentille (1).

6. Procédé selon la revendication 5, **caractérisé en ce que** le flacon rigide (30) et le corps cylindrique (32) sont fixés l'un à l'autre par vissage d'une extrémité (37) du flacon rigide (30) sur la paroi extérieure (34) du corps cylindrique (32), et de façon à assurer l'étanchéité aux liquides entre le flacon rigide (30) et la paroi extérieure (34) du corps cylindrique.

7. Dispositif d'emballage et de conservation à l'état stérile d'une lentille (1) intraoculaire hydrophile souple comprenant :
- un support d'injection (4) comprenant une extrémité d'implantation (5) par laquelle la lentille (1) peut être glissée et éjectée en vue de son implantation intraoculaire, ce support d'injection (4) étant adapté pour recevoir et porter la lentille (1) et pour pouvoir être associé à un dispositif d'injection (14) comprenant un piston pousseur (41) apte à pousser la lentille (1) vers une extrémité d'implantation (5) du support d'injection (4),
- une lentille (1) intraoculaire hydrophile souple placée sur le support d'injection (4),
- un emballage renfermant au moins la lentille (1), le support d'injection (4) et un volume de solution liquide de conservation de la lentille venant baigner la lentille (1) et la maintenant hydratée,
**caractérisé en ce que** :
- ce support d'injection (4) est adapté pour recevoir et porter la lentille (1) à plat et pour pouvoir réaliser un pliage de la lentille (1) avant que cette dernière ne soit éjectée via l'extrémité d'implantation (5),
- la lentille (1) est portée à plat sur le support d'injection (4), et immergée dans un bain de solution liquide (20, 31) de conservation contenue dans un flacon (19,30) rigide étanche aux liquides et fermé,
- l'ensemble est à l'état stérilisé.

8. Dispositif selon la revendication 7, **caractérisé en ce que** le flacon rigide (19, 30) est renfermé dans une enveloppe d'emballage (25, 48) externe compatible avec une stérilisation à la vapeur.

9. Dispositif selon l'une des revendications 7 ou 8, **caractérisé en ce que** le support d'injection (4) est adapté pour réaliser un pliage par simple mouvement de translation imparti à la lentille (1) lorsque cette dernière est poussée vers l'extrémité d'implantation (5).

10. Dispositif selon l'une des revendications 7 à 9, **caractérisé en ce que** le support d'injection (4) est porté amovible par un bouchon (17) de fermeture du flacon rigide (19).

11. Dispositif selon l'une des revendications 7 à 9, **caractérisé en ce que** le support d'injection (4) est associé à un dispositif d'injection (14) comprenant un corps cylindrique (32) creux recevant le piston pousseur (41) adapté pour pouvoir coulisser de façon étanche dans le corps cylindrique (32), et **en ce que** le flacon rigide (30) et le corps cylindrique (32) sont adaptés pour pouvoir être fixés l'un à l'autre rigidement, de façon étanche, le support d'injection (4) s'étendant dans la solution liquide de conservation dans le flacon rigide (30), mais de façon à pouvoir être dissociés l'un de l'autre en vue de l'utilisation du dispositif d'injection (14) pour l'implantation de la lentille (1).

12. Dispositif selon la revendication 11, **caractérisé en ce que** le flacon rigide (30) et le corps cylindrique (32) sont fixés l'un à l'autre par vissage d'une extrémité (37) du flacon rigide (30) sur la paroi extérieure (34) du corps cylindrique (32), et de façon à assurer l'étanchéité aux liquides entre le flacon rigide (30) et la paroi extérieure (34) du corps cylindrique (32).

13. Dispositif selon l'une des revendications 11 ou 12, **caractérisé en ce qu'**il comprend des moyens (62) formant butée axiale empêchant l'extraction intempestive du piston pousseur (41) hors du corps cylindrique creux (32).

14. Dispositif selon la revendication 13, **caractérisé en ce que** le corps cylindrique creux (32) est adapté pour former la butée d'extrémité axiale (62) empêchant l'extraction intempestive du piston pousseur (41) hors du corps cylindrique creux (32).

15. Dispositif selon la revendication 14, **caractérisé en ce qu'**il comprend un joint d'étanchéité (63) adapté pour être interposé entre la butée d'extrémité axiale (62) du corps cylindrique creux (32), et un bloc d'étanchéité (45) du piston pousseur (41) en position extrême rétracté dans le corps cylindrique creux (32).

16. Dispositif selon l'une des revendications 11 à 15, **caractérisé en ce qu'**il est doté de moyens de verrouillage déverrouillables du piston pousseur (41) en position extrême rétracté dans le corps cylindrique creux (32).

17. Dispositif selon la revendication 16, **caractérisé en ce que** le piston pousseur (41) comprend une tige de manoeuvre (46) non symétrique de révolution, **en ce que** le corps cylindrique creux (32) présente une extrémité axiale (72) dotée d'une lumière (65) non symétrique de révolution, de formes conjuguées de celles de la tige de manoeuvre (46), et **en ce que** la tige de manoeuvre (46) est montée de façon à pouvoir être tournée autour de son axe longitudinal entre une position verrouillée où elle ne peut pas pénétrer dans le lumière (65) et une position déverrouillée où elle peut passer dans la lumière (65).

18. Dispositif selon l'une des revendications 7 à 17, **caractérisé en ce que** le support d'injection (4) comprend une douille d'adaptation (81) formant un logement de réception (6) de la lentille (1), cette douille (81) étant adaptée pour pouvoir porter et recevoir différents modèles de lentilles (1,1a, 1b, 1c), et pour pouvoir être montée dans une portion d'extrémité cylindrique (7) du support d'injection (4).

## Claims

1. Method for packaging a flexible hydrophilic intraocular lens (1), in which:
- the lens (1) is placed on an injection support (4) including an implantation end (5) through which the lens (1) can be slid and ejected for intraocular implantation, said injection support (4) being adapted to receive and carry the lens (1) and to be associated with an injection device (14) including a thruster piston (41) able to push the lens (1) on the injection support (4) towards the implantation end (5),
- the lens (1) and the injection support (4) are placed in a packaging enclosing a volume of liquid solution for conserving the lens (1) which bathes the lens and keeps it hydrated,
**characterised in that**:
- an injection support (4) adapted to receive and carry the lens (1) flat and to carry out folding of the lens (1) prior to ejection of the latter via the implantation end (5) is used;
- the lens (1) is placed flat on the injection support (4) and is immersed in a bath of liquid conserving solution (20, 31) contained in a liquid-tight rigid flask (19, 30) which is closed, and
- the assembly is then steam-sterilized.

2. Method according to claim 1, **characterised in that** the rigid flask (19, 30) is placed prior to sterilization in an outer packaging envelope (25, 48) compatible with steam sterilization.

3. Method according to either of claims 1 and 2, **characterised in that** an injection support (4) is used which is adapted to carry out the folding by a simple translational movement imparted to the lens (1) when the latter is pushed towards the implantation end (5).

4. Method according to any one of claims 1 to 3, **characterised in that** an injection support (4) carried removably by a stopper (17) for closing the rigid flask (19) is used.

5. Method according to any one of claims 1 to 3, **characterised in that** an injection support (4) is used which is associated with an injection device (14) including a hollow cylindrical body (32) for receiving the thruster piston (41) adapted to slide in a sealed manner in the cylindrical body (32), and **characterised in that** the rigid flask (30) and the cylindrical body (32) are adapted to be fixed rigidly and sealingly to one another, the injection support (4) extending in the liquid conserving fluid in the rigid flask (30), but to be fixed in such a way that they can be separated from one another in order to utilize the injection device (14) to implant the lens (1).

6. Method according to claim 5, **characterised in that** the rigid flask (30) and the cylindrical body (32) are fixed to one another by screwing an end (37) of the rigid flask (30) to the outer wall (34) of the cylindrical body (32) in such a way as to ensure liquid-tightness between the rigid flask (30) and the outer wall (34) of the cylindrical body.

7. Device for packaging and conserving in a sterile condition a flexible hydrophilic intraocular lens (1), comprising:
- an injection support (4) including an implantation end (5) through which the lens (1) can be slid and ejected for intraocular implantation, said injection support (4) being adapted to receive and carry the lens (1) and to be associated with an injection device (14) including a thruster piston (41) able to push the lens (1) towards an implantation end (5) of the injection support (4);
- a flexible hydrophilic intraocular lens (1) placed on the injection support (4);
- a packaging enclosing at least the lens (1), the injection support (4) and a volume of liquid solution for conserving the lens which bathes the lens (1) and keeps it hydrated,
**characterised in that**:
- the injection support (4) is adapted to receive and carry the lens (1) flat and to carry out folding of the lens (1) prior to ejection of the latter via the implantation end (5);
- the lens (1) is carried flat on the injection support (4) and immersed in a bath of liquid conserving solution (20, 31) contained in a rigid liquid-tight flask (19, 30) which is closed, and
- the assembly is in a sterilized condition.

8. Device according to claim 7, **characterised in that** the rigid flask (19, 30) is enclosed in an outer packaging envelope (25, 48) compatible with steam sterilization.

9. Device according to either of claims 7 and 8, **characterised in that** the injection support (4) is adapted to carry out the folding by a simple translational movement imparted to the lens (1) when the latter is pushed towards the implantation end (5).

10. Device according to any one of claims 7 to 9, **characterised in that** the injection support (4) is carried removably by a stopper (17) for closing the rigid flask (19).

11. Device according to any one of claims 7 to 9, **characterised in that** the injection support (4) is associated with an injection device (14) including a hollow cylindrical body (32) for receiving the thruster piston (41) adapted to slide in a sealed manner in the cylindrical body (32), and **characterised in that** the rigid flask (30) and the cylindrical body (32) are adapted to be fixed rigidly and sealingly to one another, the injection support (4) extending in the liquid conserving fluid in the rigid flask (30), but to be fixed in such a way that they can be separated from one another in order to utilize the injection device (14) to implant the lens (1).

12. Device according to claim 11, **characterised in that** the rigid flask (30) and the cylindrical body (32) are fixed to one another by screwing an end (37) of the rigid flask (30) to the outer wall (34) of the cylindrical body (32) in such a way as to ensure liquid-tightness between the rigid flask (30) and the outer wall (34) of the cylindrical body (32).

13. Device according to either of claims 11 and 12, **characterised in that** it includes means (62) forming an axial stop which prevents premature extraction of the thruster piston (41) from the hollow cylindrical body (32).

14. Device according to claim 13, **characterised in that** the hollow cylindrical body (32) is adapted to form the axial end stop (62) preventing premature extraction of the thruster piston (41) from the hollow cylindrical body (32).

15. Device according to claim 14, **characterised in that** it includes a seal (63) adapted to be interposed between the axial end stop (62) of the hollow cylindrical body (32) and a sealing block (45) of the thruster piston (41) when in its retracted end position in the hollow cylindrical body (32).

16. Device according to any one of claims 11 to 15, **characterised in that** it is provided with unlockable means for locking the thruster piston (41) in its retracted end position in the hollow cylindrical body (32).

17. Device according to claim 16, **characterised in that** the thruster piston (41) includes a non-rotationally-symmetrical operating stem (46), **characterised in that** the hollow cylindrical body (32) has an axial end (72) provided with a non-rotationally-symmetrical opening (65) having a shape matching that of the operating stem (46), and **characterised in that** the operating stem (46) is so mounted as to be able to be rotated about its longitudinal axis between a locked position in which it cannot pass through the opening (65) and an unlocked position in which it can pass through the opening (65).

18. Device according to any one of claims 7 to 17, **characterised in that** the injection support (4) includes an adapter bush (81) forming a receptacle (6) for the lens (1), the bush (81) being adapted to be able to carry and receive different models of lens (1, 1a, 1b, 1c), and to be mounted in a cylindrical end portion (7) of the injection support (4).

## Patentansprüche

1. - Verfahren zum Verpacken einer hydrophilen, flexiblen Intraokularlinse (1), bei dem:
- Die Linse (1) auf einen Injektionsträger (4) gesetzt wird, der ein Implantationsende (5) umfaßt, mit dem die Linse (1) zwecks intraokularer Implantation geschoben und ausgestoßen werden kann, wobei dieser Injektionsträger (4) so gestaltet ist, dass er die Linse (1) aufnimmt und trägt und mit einer Injektionsvorrichtung (14) verbunden werden kann, die einen Schubkolben (41) umfaßt, der geeignet ist, die Linse (1) auf dem Injektionsträger (4) zum Implantationsende (5) zu schieben,
- Die Linse (1) und der Injektionsträger (4) werden in eine Verpackung gegeben, die ein Volumen Lösungsflüssigkeit zur Aufbewahrung der Linse (1) umschließt, in der die Linse badet und somit hydratisiert gehalten wird,
**dadurch gekennzeichnet, daß**:
- Es ein Injektionsträger (4) benutzt wird, der dafür angepaßt ist, die Linse (1) aufzunehmen und flachliegend zu tragen, um eine Faltung der Linse (1) ausführen zu können, bevor letztere vermittels des Implantationsendes (5) ausgestoßen wird,
- Die Linse (1) flachliegend auf den Injektionsträger (4) gesetzt wird, und sie in ein Bad mit Lösungsflüssigkeit (20, 31) zur Aufbewahrung eingetaucht wird, das in einem steifen gegen die Flüssigkeiten dichten Fläschchen (19, 30) enthalten ist, das wieder geschlossen wird,
- Das Ganze wird dann unter Dampf sterilisiert.

2. - Verfahren nach Anspruch 1, **dadurch gekennzeichnet, daß** das steife Fläschchen (19, 30) vor der Sterilisierung in eine äußere Verpackungshülle (25, 48) gesteckt wird, die mit einer Dampfsterilisation kompatibel ist.

3. - Verfahren nach einem der vorstehenden Ansprüche 1 oder 2, **dadurch gekennzeichnet, daß** ein Injektionsträger (4) benutzt wird, der geeignet ist, eine Faltung durch einfache auf die Linse (1) übertragene Querbewegung auszuführen, wenn letztere zum Implantationsende (5) gedrückt wird.

4. - Verfahren nach einem der vorstehenden Ansprüche 1 bis 3, **dadurch gekennzeichnet, daß** ein Injektionsträger (4) benutzt wird, der abnehmbar von einem Verschluß (17) des steifen Fläschchens (19) getragen wird.

5. - Verfahren nach einem der vorstehenden Ansprüche 1 bis 3, **dadurch gekennzeichnet, daß** ein Injektionsträger (4) benutzt wird, der mit einer Injektionsvorrichtung (14) verbunden ist, die einen zylinderförmigen Hohlkörper (32) umfaßt, der den Schubkolben (41) aufnimmt, der dafür geeignet ist, im zylinderförmigen Körper (32) dicht zu gleiten, und daß das steife Fläschchen (30) und der zylinderförmige Körper (32) so angepaßt sind, daß sie steif und dicht aneinander befestigt werden können, wobei sich der Injektionsträger (4) in der Lösungsflüssigkeit zur Aufbewahrung im steifen Fläschchen (30) ausbreitet, jedoch so, daß sie für die Benutzung der Injektionsvorrichtung (14) zwecks Implantation der Linse (1) voneinander getrennt werden können.

6. - Verfahren nach Anspruch 5, **dadurch gekennzeichnet, daß** das steife Fläschchen (30) und der zylinderförmige Körper (32) durch Verschrauben eines Endes (37) des steifen Fläschchens (30) auf die Außenwand (34) des zylinderförmigen Körpers (32) aneinander befestigt sind, damit die Dichtheit gegen die Flüssigkeiten zwischen dem steifen Fläschchen (30) und der Außenwand (34) des zylinderförmigen Körpers gewährleistet ist.

7. - Vorrichtung zur Verpackung und sterilen Aufbewahrung einer hydrophilen, flexiblen Intraokularlinse (1), die umfaßt:
- Einen Injektionsträger (4), der ein Implantationsende (5) umfaßt, mit dem die Linse (1) zwecks ihrer intraokularen Implantation geschoben und ausgestoßen werden kann, wobei dieser Injektionsträger (4) so gestaltet ist, daß er die Linse (1) aufnimmt und trägt, und daß er mit einer Injektionsvorrichtung (14) verbunden werden kann, die einen Schubkolben (41) umfaßt, der geeignet ist, die Linse (1) zu einem Implantationsende (5) des Injektionsträgers (4) zu schieben,
- Eine hydrophile, flexible Intraokularlinse (1) auf einem Injektionsträger (4),
- Eine Verpackung, die mindestens die Linse (1), den Injektionsträger (4) und ein Volumen Lösungsflüssigkeit zur Aufbewahrung der Linse enthält, in der die Linse (1) badet und somit hydratisiert gehalten wird,
**dadurch gekennzeichnet, daß**:
- Dieser Injektionsträger (4) dafür angepaßt ist, die Linse (1) aufzunehmen und flachliegend zu tragen, um eine Faltung der Linse (1) ausführen zu können, bevor letztere vermittels des Implantationsendes (5) ausgestoßen wird,
- Die Linse (1) flachliegend auf dem Injektionsträger (4) getragen wird, und sie in ein Bad mit Lösungsflüssigkeit (20, 31) zur Aufbewahrung eingetaucht wird, das in einem steifen gegen die Flüssigkeiten dichten und geschlossenen Fläschchen (19, 30) enthalten ist,
- Das Ganze ist im sterilen Zustand.

8. - Vorrichtung nach Anspruch 7, **dadurch gekennzeichnet, daß** das steife Fläschchen (19, 30) in eine äußere Verpackungshülle (25, 48) eingeschlossen wird, die mit einer Dampfsterilisation kompatibel ist.

9. - Vorrichtung nach einem der vorstehenden Ansprüche 7 oder 8, **dadurch gekennzeichnet, daß** der Injektionsträger (4) geeignet ist, eine Faltung durch einfache auf die Linse (1) übertragene Querbewegung auszuführen, wenn letztere zum Implantationsende (5) gedrückt wird.

10. - Vorrichtung nach einem der vorstehenden Ansprüche 7 bis 9, **dadurch gekennzeichnet, daß** der Injektionsträger (4) abnehmbar von einem Verschluß (17) des steifen Fläschchens (19) getragen wird.

11. - Vorrichtung nach einem der vorstehenden Ansprüche 7 bis 9, **dadurch gekennzeichnet, daß** der Injektionsträger (4) mit einer Injektionsvorrichtung (14) verbunden ist, die einen zylinderförmigen Hohlkörper (32) umfaßt, der den Schubkolben (41) aufnimmt, der dafür geeignet ist, im zylinderförmigen Körper (32) dicht zu gleiten, und daß das steife Fläschchen (30) und der zylinderförmige Körper (32) so angepaßt sind, daß sie steif und dicht aneinander befestigt werden können, wobei sich der Injektionsträger (4) in der flüssigen Lösung zur Aufbewahrung im steifen Fläschchen (30) ausbreitet, jedoch so, daß sie für die Benutzung der Injektionsvorrichtung (14) zwecks Implantation der Linse (1) voneinander getrennt werden können.

12. - Vorrichtung nach Anspruch 11, **dadurch gekennzeichnet, daß** das steife Fläschchen (30) und der zylinderförmige Körper (32) durch Verschrauben eines Endes (37) des steifen Fläschchens (30) auf die Außenwand (34) des zylinderförmigen Körpers (32) aneinander befestigt sind, damit die Dichtheit gegen die Flüssigkeiten zwischen dem steifen Fläschchen (30) und der Außenwand (34) des zylinderförmigen Körpers (32) gewährleistet ist.

13. - Vorrichtung nach einem der vorstehenden Ansprüche 11 oder 12, **dadurch gekennzeichnet, daß** sie Mittel (62) umfaßt, die einen axialen Anschlag bilden, der die unbeabsichtigte Extraktion des Schubkolbens (41) aus dem zylinderförmigen Hohlkörper (32) verhindern.

14. - Vorrichtung nach Anspruch 13, **dadurch gekennzeichnet, daß** der zylinderförmige Hohlkörper so gestaltet ist, daß er die unbeabsichtigte Extraktion des Schubkolbens (41) aus dem zylinderförmigen Hohlkörper (32) verhindert.

15. - Vorrichtung nach Anspruch 14, **dadurch gekennzeichnet, daß** sie eine Dichtung (63) umfaßt, die geeignet ist, zwischen den axialen Endanschlag (62) des zylinderförmigen Hohlkörpers (32) und einem Dichtblock (45) des Schubkolbens (41) in Endposition in den zylinderförmigen Hohlkörper (32) zurückgezogen eingesetzt werden kann.

16. - Vorrichtung nach einem der vorstehenden Ansprüche 11 bis 15, **dadurch gekennzeichnet, daß** sie mit entriegelbaren Verriegelungsmitteln des Schubkolbens (41) in Endposition in den zylinderförmigen Hohlkörper (32) zurückgezogen versehen ist.

17. - Vorrichtung nach Anspruch 16, **dadurch gekennzeichnet, daß** der Schubkolben (41) eine nicht symmetrische Bedienungsspindel (46) zur Drehung umfaßt, daß der zylinderförmige Hohlkörper (32) ein axiales Ende (72) aufweist, das mit einer nicht symmetrischen Öffnung (65) zur Drehung mit von der Bedienungsspindel (46) abgeleiteten Formen versehen ist, daß die Bedienungsspindel (46) so montiert ist, daß sie um ihre Längsachse zwischen einer verriegelten Position, in der sie nicht in die Öffnung (65) eintreten kann, und einer entriegelten Position gedreht werden kann, in der sie in die Öffnung (65) eintreten kann.

18. - Vorrichtung nach einem der vorstehenden Ansprüche 7 bis 17, **dadurch gekennzeichnet, daß** der Injektionsträger (4) eine Paßhülse (81) umfaßt, die eine Aufnahme (6) für die Linse (1) bildet, wobei diese Hülse (81) angepaßt ist, daß sie verschiedene Linsenmodelle (1, 1a, 1b, 1c) tragen und aufnehmen kann, und daß sie in einen zylinderförmigen Endteil (7) des Injektionsträgers (4) montiert werden kann.
